# NEUE EUROPÄISCHE PATENTSCHRIFT

(11) **EP 1 515 702 B3**
(45) Veröffentlichungstag dieser Patentschrift: **12.01.2022**
(45) Hinweis auf die Patenterteilung: 15.10.2008
(21) Anmeldenummer: 03735628.4
(22) Anmeldetag: 16.06.2003
(51) Int. Cl.: A61K 9/20, A61K 9/48

(54) **GEGEN MISSBRAUCH GESICHERTE DARREICHUNGSFORM**
ABUSE-PROTECTED ADMINISTRATION FORM
FORME D'ADMINISTRATION SECURISEE CONTRE L'USAGE ABUSIF

(30) Priorität: 17.06.2002 DE 10227077; 25.10.2002 DE 10250083
(43) Veröffentlichungstag der Anmeldung: 23.03.2005
(73) Patentinhaber: Grünenthal GmbH, 52078 Aachen (DE)
(72) Erfinder: BARTHOLOMÄUS, Johannes, 52080 Aachen (DE); KUGELMANN, Heinrich, 52068 Aachen (DE)
(74) Vertreter: Kutzenberger, Helga
(86) Internationale Anmeldenummer: PCT/EP2003/006314
(87) Internationale Veröffentlichungsnummer: WO 2003/105808

(56) Entgegenhaltungen:
- WO-A-95/20947
- WO-A-03/013476
- US-A- 3 980 766
- US-A- 4 070 494

## Beschreibung

Die vorliegende Erfindung betrifft eine feste Darreichungsform mit verminderten parenteralen Mißbrauch enthaltend neben (1R*,2R*)-3-(3-Dimethylamino-1-ethyl-2-methylpropyl)-phenol oder einem physiologisch verträglichen Salz davon wenigstens ein solches viskositätserhöhendes Mittel in solchen Mengen, dass bei einer mit Hilfe einer notwendigen Mindestmenge an wäßriger Flüssigkeit Extraktion ein vorzugsweise noch nadelgängiges Gel gebildet wird, welches aber selbst beim Einbringen in eine weitere Menge einer wäßrigen Flüssigkeit visuell unterscheidbar bleibt.

Eine Vielzahl von pharmazeutischen Wirkstoffen weist neben einer ausgezeichneten Wirksamkeit auf ihrem betreffenden Anwendungsgebiet auch ein Mißbrauchspotential auf, d.h. sie können von einem Mißbraucher eingesetzt werden, um Wirkungen herbeizuführen, die nicht ihrem Bestimmungszweck entsprechen. So werden beispielsweise Opiate, die eine exzellente Wirksamkeit bei der Bekämpfung von starken bis sehr starken Schmerzen zeigen, von Mißbrauchern häufig zum Einleiten rauschartiger, euphorisierender Zustände verwendet.

Darreichungsformen, die Wirkstoffe mit Mißbrauchspotential enthalten, führen üblicherweise selbst bei einer oralen mißbräuchlich hohen Menge nicht zu dem vom Mißbraucher gewünschten Ergebnis, nämlich einen raschen Kick, da die Wirkstoffe im Blut nur langsam anfluten. Um dennoch den gewünschten Effekt zu erzielen und den Mißbrauch zu ermöglichen, werden die entsprechenden Darreichungsformen vom Mißbraucher zerkleinert, z. B. gemörsert, und der Wirkstoff aus dem durch Zerkleinerung der Darreichungsform erhaltenen Pulver mit Hilfe einer vorzugsweise wäßrigen Flüssigkeit, vorzugsweise in notwendigen Mindestmengen, extrahiert und die resultierende Lösung, ggf. nach Filtration durch Watte oder Zellstoff, parenteral, insbesondere intravenös, appliziert. Wegen dieser parenteralen Verabreichung werden nur möglichst geringe Mengen einer wäßrigen Flüssigkeit zur Extraktion eingesetzt, insbesondere um eine möglichst kleines Injektionsvolumen mit Wirkstoff zur Verfügung zu haben, das zu dem gewünschten raschen Kick führt. Damit kommt es bei der parenteralen Verabreichung gegenüber der oralen Applikation weiter eher zur einem beschleunigten Anfluten des Wirkstoffes mit dem vom Mißbraucher gewünschten Ergebnis.

Zur Verhinderung dieser Form des Mißbrauchs wurde in der US 4,070,494 vorgeschlagen, der Extraktion eines Wirkstoffes aus einer Darreichungsform durch Zusatz eines quellbaren Mittels vorzubeugen. Dieses quillt bei der Zugabe von Wasser auf und bewirkt, dass nur eine geringe Menge an Wirkstoff-haltiger, vom Mißbraucher parenteral applizierbare Flüssigkeit erhalten wird. Der gequollene Hauptteil der Darreichungsform ist nicht applizierbar.

Ein entsprechender Ansatz zur Verhinderung des parenteralen Mißbrauchs liegt auch der in der WO 95120947 offenbarten Mehrschichttablette zugrunde, die jeweils den Wirkstoff mit Mißbrauchspotential und einen oder mehrere Gelbildner in verschiedenen Schichten aufweist.

Gemäß diesen Lehren des Standes der Technik werden die viskositätserhöhenden Mittel in solchen Mengen zugesetzt, dass das entsprechende Gel mit Hilfe von üblichen Injektionsnadeln nicht verabreicht werden kann.

Die Aufgabe der vorliegenden Erfindung bestand darin, eine Darreichungsform mit zumindest verminderten Mißbrauchspotential für Wirkstoffe mit einem solchen Potential zur Verfügung zu stellen, die einen vorzugsweise noch möglichen parenteralen, insbesondere intravenösen Mißbrauch der Wirkstoffe, verhindert wird.

Diese Aufgabe wurde durch Bereitstellung der erfindungsgemäßen, festen Darreichungsform gemäss Anspruch 1 gelöst.

Visuelle Unterscheidbarkeit im Sinne der vorliegenden Erfindung bedeutet, dass das mit Hilfe einer notwendigen Mindestmenge an wäßriger Flüssigkeit durch Extraktion aus der Darreichungsform gebildete, Wirkstoff-haltige Gel beim Einbringen mit einer Injektionsnadel mit einem Durchmesser von 0,9 mm, in eine weitere Menge wäßriger Flüssigkeit von 37°C im wesentlichen unlöslich und zusammenhängend bleibt und nicht auf einfache Weise so dispergiert werden kann, dass eine parenterale, insbesondere intravenöse, gefahrlose Applikation möglich ist. Vorzugsweise beträgt die Dauer der visuellen Unterscheidbarkeit wenigstens eine Minute, vorzugsweise mindestens 10 min.

Die Viskositätserhöhung des Extrakts mit Hilfe ausgewähltem viskositätserhöhenden Mittel führt dazu, dass dessen Nadelgängigkeit bzw. Spritzbarkeit zwar erschwert wird, aber noch möglich ist. Des weiteren führt sie dazu, dass der erhaltene Extrakt bzw. das Gel beim Einbringen bei 37°C in eine weitere Menge wäßriger Flüssigkeit, z. B. auch durch Einspritzen in Blut, zunächst in Form eines weitgehend zusammenhängenden Fadens erhalten bleibt, der zwar durch mechanische Einwirkung in kleinere Bruchstücke zerteilt, nicht aber so dispergiert oder sogar gelöst werden kann, dass eine parenterale, insbesondere intravenöse, Applikation gefahrlos möglich ist. Eine intravenöse Applikation eines entsprechenden Extraktes würde daher mit großer Wahrscheinlichkeit zur Verstopfung von Gefäßen, verbunden mit schweren Embolien bis hin zum Tod des Mißbrauchers führen.

Unter Nadelgängigkeit des mit Hilfe einer notwendigen Mindestmenge an einer wässrigen Flüssigkeit, vorzugsweise Wasser, aus der erfindungsgemäßen Darreichungsform gewonnene Extrakt bzw. Gel wird im Sinne der vorliegenden Erfindung verstanden, dass das so gebildete Gel durch eine Injektionsnadel mit einem Durchmesser von 2 mm, vorzugsweise 1,5 mm, besonders bevorzugt 0,6 mm, noch aufgezogen werden kann und auch daraus wieder gespritzt werden kann. Der pharmazeutische Wirkstoff (1R*,2R*)-3-(3-Dimethylamino-1-ethyl-2-methyl-propyl)-phenol ist dem Fachmann, ebenso wie dessen einzusetzende Mengen und Verfahren zu dessen Herstellung, an sich bekannt und kann als solcher in Form entsprechender Derivate, insbesondere Ester oder Ether, oder jeweils in Form entsprechender physiologisch verträglicher Verbindungen, insbesondere in Form seiner Salze oder Solvate, in der erfindungsgemäßen Darreichungsform vorliegen. Die erfindungsgemäße Darreichungsform eignet sich auch für die Verabreichung von mehreren Wirkstoffen. Vorzugsweise wird sie zur Verabreichung eines Wirkstoffs eingesetzt.

Die erfindungsgemäße Darreichungsform eignet sich zur Verhinderung des Mißbrauchs von (1R*,2R*)-3-(3-Dimethylamino-1-ethyl-2-methyl-propyl)-phenol, dessen entsprechende Derivate, insbesondere Ester oder Ether, und dessen physiologisch verträgliche Verbindungen, insbesondere dessen Salze und Solvate.

Die Verbindung (1R*,2R*)-3-(3-Dimethylamino-1-ethyl-2-methyl-propyl)-phenol, deren physiologisch verträglichen Verbindungen, insbesondere deren Hydrochloride sowie Verfahren zu ihrer Herstellung sind beispielsweise aus aus EP-A-693475 bekannt. Die entsprechende Beschreibung wird hiermit als Referenz eingeführt und gilt als Teil der Offenbarung.

Zur Überprüfung, ob ein viskositätserhöhendes Mittel zur Anwendung in der erfindungsgemäßen Darreichungsform geeignet ist, wird dieses zunächst in einer entsprechenden Darreichungsform in solchen Mengen formuliert, dass keine nennenswerte (± 5%) Beeinflussung der Wirkstoff-Freisetzung gegenüber einer Darreichungsform ohne viskositätserhöhendes Mittel. Die entsprechende Darreichungsform wird außerdem zerkleinert, vorzugsweise gemörsert, und mit 10 ml Wasser bei 25°C extrahiert. Bildet sich weiterhin hierbei ein Gel, welches den vobenstehend genannten Bedingungen genügt, eignet sich das entsprechende viskositätserhöhende Mittel zur Herstellung einer erfindungsgemäßen Darreichungsform.

Vorzugsweise kommen eine oder mehrere viskositätserhöhende Mittel in der erfindungsgemäßen Darreichungsform zum Einsatz, die ausgewählt sind aus der Gruppe bestehend aus mikrokristalliner Cellulose mit 11 Gew.-% Carboxymethylcellulose-Natrium (Avicel ^{®} RC 591), Carboxymethylcellulose-Natrium (Blanose ^{®}, CMC-Na C300P ^{®}, Frimulsion BLC-5 ^{®}, Tylose C300 P ^{®}), Polyacrylsäure (Carbopol ^{®} 980 NF, Carbopol ^{®} 981), Johannisbrotkemmehl (Cesagum ^{®} LA-200, Cesagum ^{®} LID/150, Cesagum ^{®} LN-1), Pectine, vorzugsweise aus Citrusfrüchten oder Äpfeln (Cesapectin ^{®} HM Medium Rapid Set), Wachsmaisstärke (C*Gel 04201 ^{®}), Natriumalginat (Frimulsion ALG (E401) ^{®}) Guarkemmehl (Frimulsion BM ^{®}, Polygum 26/1-75 ^{®}), lota-Carrageen (Frimulsion D021 ^{®}), Karaya Gummi, Gellangummi (Kelcogel F ^{®}, Kelcogel LT100 ^{®}), Galaktomannan (Meyprogat 150 ^{®}), Tarakemmehl (Polygum 43/1 ^{®}), Propylenglykoalginat (Protanal-Ester SD-LB ^{®}), NatriumHyaluronat, Tragant, Taragummi (Vidogum SP 200 ^{®}), fermentiertes Polysaccharid- Welan Gum (K1A96), Xanthane wie Xanthan-Gummi (Xantural 180 ^{®}). Die in Klammern angegebenen Bezeichnungen sind Beispiele für Handelsnamen, unter denen die jeweiligen Materialien am Markt geführt sind.

Im allgemeinen ist eine Menge von 0,1 bis 25 Gew.-%, vorzugsweise 0,5 bis 15 Gew.-%, besonders bevorzugt 1-10 Gew.-% der viskositätserhöhenden Mittel bezogen auf die Gesamtformulierung, ausreichend, um die vorstehend genannten Bedingungen zu erfüllen. Die viskositätserhöhenden Mittel liegen in der erfindungsgemäßen Darreichungsform bevorzugt in Mengen von ≥ 5 mg, besonders bevorzugt ≥ 10 mg pro Darreichungsform, d.h. pro Dosiereinheit vor.

In einer besonders bevorzugten Ausführungsform der vorliegenden Erfindung kommen solche viskositätserhöhende Mittel zum Einsatz, die neben den vorstehend genannten Bedingungen auch bei der Extraktion aus der Darreichungsform mit der notwendigen Mindestmenge an wäßriger Flüssigkeit ein Gel bilden, das Luftblasen einschließt. Die so erhaltenen Gele zeichnen sich durch ein trübes Erscheinungsbild aus, durch das der potentielle Mißbraucher zusätzlich optisch gewarnt und von dessen parenteraler Applikation abgehalten wird.

(1R*,2R*)-3-(3-Dimethylamino-1-ethyl-2-methyl-propyl)-phenol und die viskositätserhöhenden Mittel sowie ggf. physiologisch verträgliche Hilfsstoffe können nach üblichen, dem Fachmann bekannten Methoden zu der erfindungsgemäßen Darreichungsform formuliert werden. Entsprechende Methoden zur Formulierung der erfindungsgemäßen Darreichungsform sind dem Fachmann an sich bekannt, beispielsweise aus " Coated Pharmaceutical Dosage Form - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers. Die entsprechende Literaturbeschreibung wird hiermit als Referenz eingeführt und gilt als Teil der Offenbarung. Überraschenderweise ist es durch die erfinderische Auswahl der viskositätserhöhenden Mittel möglich, den Wirkstoff und die viskositätserhöhenden Mittel ohne räumliche Trennung voneinander in der erfindungsgemäßen Darreichungsform zu kombinieren, ohne dass die Freisetzung des Wirkstoffs bei bestimmungsgemäßer Applikation der Darreichungsform gegenüber einer entsprechenden Darreichungsform, die das viskositätserhöhende Mittel nicht aufweist, beeinträchtigt wird. Selbstverständlich ist es aber auch möglich, die viskositätserhöhenden Mittel und den Wirkstoff in räumlich voneinander getrennter Anordnung in der Darreichungsform zu kombinieren.

Die erfindungsgemäßen, festen, gegen parenteralen Mißbrauch geschützten Darreichungsformen eignen sich bevorzugt zur oralen oder rektalen Applikation, besonders bevorzugt zur oralen Applikation.

Sofern die erfindungsgemäße Darreichungsform zur rektalen Applikation vorgesehen ist, liegt sie bevorzugt in Form eines Suppositoriums vor.

Ist die erfindungsgemäße Darreichungsform zur oralen Applikation vorgesehen, liegt sie vorzugsweise in Form einer Tablette, einer Kapsel oder in Form eines oralen osmotischen therapeutischen Systems (OROS) vor.

Orale osmotische therapeutische Systeme sowie geeignete Materialien und Verfahren zu ihrer Herstellung sind dem Fachmann an sich bekannt, beispielsweise aus US 4,612,008, US 4,765,989 und US 4,783,337. Die entsprechenden Beschreibungen werden hiermit als Referenz eingeführt und gelten als Teil der Offenbarung. Das entsprechende orale osmotische therapeutische System kann bevorzugt als Ein- oder Zweikammersystem, jeweils mit einschichtigem oder mehrschichtigem Aufbau vorliegen. Vorzugsweise besteht die Push-Schicht in diesen Systemen, d.h. die Schicht, die durch ihr Quellen den osmotischen Druck erzeugt, durch den die darüber liegende Schicht aus dem System herausgedrückt wird, zumindest teilweise aus den erfindungsgemäß zum Einsatz kommenden viskositätserhöhenden Mitteln.

In einer weiteren bevorzugten Ausführungsform der vorliegenden Erfindung liegt die oral applizierbare, erfindungsgemäße Darreichungsform in multipartikulärer Form enthaltend jeweils die Gesamtmischung aus Wirkstoff und viskositätserhöhendem Mittel, vorzugsweise in Form von Mikrotabletten, Mikrokapseln, Mikropellets, Granulaten, Sphäroiden, Perlen oder Pellets, vorzugsweise in Kapseln abgefüllt oder zu Tabletten verpreßt, vor. Vorzugsweise weisen die mulitpartikulären Formen eine Größe im Bereich von 0,1 bis 3 mm, besonders bevorzugt im Bereich von 0,5 bis 2 mm auf.

Die erfindungsgemäßen Darreichungsform kann vorzugsweise auch den Wirkstoff, abgemischt mit dem viskositätserhöhenden Mittel, zumindest teilweise in retardierter Form aufweisen, wobei die Retardierung mit Hilfe von üblichen, dem Fachmann bekannten Materialien und Verfahren erzielt werden kann, beispielsweise durch Einbetten des Wirkstoffes in eine retardierende Matrix oder durch das Aufbringen eines oder mehrerer retardierender Überzüge.

Bevorzugt kann die retardierte Freisetzung des Wirkstoffes auch durch die gezielte Auswahl eines oder mehrerer der vorstehend genannten viskositätserhöhenden Mittel in geeigneten Mengen als Matrixmaterial erreicht werden. Das für die jeweils gewünschte Freisetzung des Wirkstoffes geeignete Mittel und dessen Menge kann der Fachmann durch einfache Vorversuche ermitteln, wobei selbstverständlich darauf zu achten ist, dass es beim Versuch des Mißbrauchs der resultierenden Darreichungsform, wie vorstehend beschrieben, zu einer Gelbildung kommt.

In jedem Fall ist darauf zu achten, dass die retardierenden Hilfsstoffe, ebenso wie weitere, ggf. vorhandene Hilfsstoffe nicht mit der Gelbildung interferieren oder die Stabilität des gebildeten Gels beeinträchtigen.

Sofern die erfindungsgemäße Darreichungsform zur oralen Applikation vorgesehen ist, kann sie auch einen magensaftresistenten Überzug aufweisen, der sich in Abhängigkeit vom pH-Wert der Freisetzungsumgebung auflöst.

Durch diesen Überzug wird erreicht, dass die erfindungsgemäße Darreichungsform bei bestimmungsgemäßer Applikation den Magentrakt unaufgelöst passiert und der Wirkstoff erst im Darmtrakt zur Freisetzung gelangt. Vorzugsweise löst sich der magensaftresistente Überzug bei einem pH-Wert zwischen 5 und 7,5 auf

Entsprechende Materialien und Verfahren zur Retardierung von Wirkstoffen sowie zum Aufbringen magensaftresistenter Überzüge sind dem Fachmann an sich bekannt, beispielsweise aus " Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers. Die entsprechende Literaturbeschreibung wird hiermit als Referenz eingeführt und gilt als Teil der Offenbarung. In einer weiteren bevorzugten Ausfuhrungsform enthält die erfindungsgemäße Darreichungsform den Wirkstoff neben seiner retardierten Form auch in seiner unretardierten Form. Durch Kombination mit dem sofort freigesetzten Wirkstoff läßt sich eine hohe Initialdosis zur schnellen Schmerzlinderung erzielen. Die langsame Freisetzung aus der retardierten Form verhindert dann ein rasches Abklingen der Wirkung.

Im folgenden wird die Erfindung anhand von Beispielen erläutert. Diese Erläuterungen sind lediglich beispielhaft und schränken den allgemeinen Erfindungsgedanken nicht ein.

### Beispiele:

### Beispiel 1

Matrixtabletten mit folgender Zusammensetzung pro Tablette

| | |
|---|---|
| (-)-(1R,2R)-3-(3-Dimethylamino-1-ethyl-2-methyl-propyl)phenol-Hydrochlorid | 100 mg |
| Hydroxypropylmethylcellulose (Metolose 90 SH 100.000 von Fa. Shinetsu), 100.000 mPa·s | 70 mg |
| Xanthan, NF | 10 mg |
| Mikrokristalline Cellulose (Avicel PH 102 von Fa. FMC) | 123 mg |
| Hochdisperses Siliciumdioxid | 4 mg |
| Magnesiumstearat | 3 mg |
| Gesamtmenge | 310 mg |

wurden in einer Ansatzgröße von 1000 Tabletten in folgender Weise hergestellt: Alle Bestandteile wurden eingewogen und auf einer Siebmaschine Quadro Comil U10 unter Verwendung einer Siebgröße von 0,813 mm gesiebt, in einem Containermischer (Bohle LM 40) 15 min ± 15 s bei einer Drehzahl von 20 ± 1 U/min gemischt und auf einer Korsch EK0 Exzenterpresse zu drageegewölbten Tabletten mit einem Durchmesser von 10 mm, einem Wölbungsradius von 8 mm und einem mittleren Tablettengewicht von 310 mg gepreßt.

Die Freisetzung in vitro wurde bestimmt unter Anwendung der Ph. Eur. Paddle Method bei 75 U/min in 900 ml Puffer pH 6,8 nach Ph. Eur. bei 37 °C und mit UVspektrometrischem Nachweis und ist in folgender Tabelle nebst eines Vergleiches mit einer entsprechenden Tablette mit 80 mg Hydroxypropylmethylcellulose ("HPMC") ohne Xanthan-Zusatz wiedergegeben.

| **Zeit [min]** | **Freigesetzte Gesamtmenge des Wirkstoffs [%] aus Tabletten gemäß Beispiel 1 (70 mg HPMC +10 mg Xanthan(** | **Freigesetzte Gesamtmenge des Wirkstoffs [%] aus Tabletten mit 80 mg HPMC (ohne Xanthan)** |
|---|---|---|
| 0 | 0 | 0 |
| 30 | 19 | 18 |
| 240 | 62 | 59 |
| 480 | 83 | 80 |
| 600 | 88 | 87 |
| 720 | 93 | 93 |

Eine der Xanthan-haltigen Tabletten wurde gemörsert und mit 10 ml Wasser geschüttelt. Es bildete sich eine viskose, trübe Suspension. Nach Absetzen der groben, festen Bestandteile der Suspension wurde das gebildete Gel in eine Spritze mit einer Nadel mit 0,9 mm Durchmesser aufgezogen. Das aufgezogene Gel wird in 37°C warmes Wasser gespritzt, wobei deutlich Fäden mit dem Durchmesser der Nadel erkennbar blieben, die sich nicht mit dem Wasser mischten. Unter Rühren könnten die Fäden zwar geteilt, aber nicht gelöst werden; wobei die Bruchstücke der Fäden mit dem bloßen Auge erkennbar bleiben. Bei Injektion eines derartigen Extraktes in Blutgefäße käme es zu Verstopfungen in den Gefäßen.

### Beispiel 2 (Vergleichsbeispiel wegen sehr schlechter Nadelgängigkeit)

Matrixtabletten mit folgender Zusammensetzung pro Tablette

| | | |
|---|---|---|
| (-)-(R,2R)-3-(3-Dimethylamino-1-ethyl-2-methyl-propyl)phenol-Hydrochlorid | 100 mg | |
| Hydroxypropylmethylcellulose (Metolose 90 SH 100.000 von Fa. Shinetsu), 100.000 mPa·s | 40 mg | |
| Xanthan, NF | 40 mg | 12 % |
| Mikrokristalline Cellulose (Avicel PH 102 von Fa. FMC) | 123 mg | |
| Hochdisperses Siliciumdioxid | 4 mg | |
| Magnesiumstearat | 3 mg | |
| Gesamtmenge | 310 mg | |

wurden wie in Beispiel 1 angegeben hergestellt und bezüglich ihrer Freisetzung untersucht.

| **Zeit [min]** | **Freigesetzte Gesamtmenge des Wirkstoffs [%]** |
|---|---|
| 0 | 0 |
| 30 | 19 |
| 240 | 61 |
| 480 | 81 |
| 600 | 87 |
| 720 | 91 |

Eine der Tabletten wurde gemörsert und mit 10 ml Wasser geschüttelt. Es bildete sich eine viskose, trübe Suspension, deren Viskosität höher als in Beispiel 1 war; mit eingeschlossenen Luftblasen. Nach Absetzen der groben, festen Bestandteile der Suspension wurde das Gel in eine Spritze mit einer Nadel mit 0,9 mm Durchmesser aufgezogen. Das aufgezogene Gel wurde in 37° C warmes Wasser gespritzt, wobei deutlich Fäden mit dem Durchmesser der Nadel erkennbar wurden, die sich nicht mit dem Wasser mischten. Unter Rühren konnten die Fäden zwar geteilt, aber nicht gelöst werden; wobei die Bruchstücke der Fäden mit dem bloßen Auge erkennbar blieben. Bei Injektion eines derartigen Geles in Blutgefäße käme es zu Verstopfungen in den Gefäßen.

### Beispiel 3

Matrixtabletten mit folgender Zusammensetzung pro Tablette

| | |
|---|---|
| (-)-(1R,2R)-3-(3-Dimethylamino-1-ethyl-2-methyl-propyl)phenol-Hydrochlorid | 100 mg |
| Xanthan, NF | 80 mg |
| Mikrokristalline Cellulose (Avicel PH 102 von Fa. FMC) | 123 mg |
| Hochdisperses Siliciumdioxid | 4 mg |
| Magnesiumstearat | 3 mg |
| Gesamtmenge | 310 mg |

wurden wie in Beipiel 1 angegeben hergestellt.

Eine dieser Tabletten wurden gemörsert und mit 10 ml Wasser geschüttelt. Es bildete sich eine viskose, trübe Suspension, deren Viskosität höher als in Beispiel 1 und und Luftblasen eingeschlossen hatte. Nach Absetzen der groben, festen Bestandteile der Suspension wurde das Gel in eine Spritze mit einer Nadel mit 0,9 mm Durchmesser aufgezogen. Das aufgezogene Gel wurde in 37°C warmes Wasser gespritzt, wobei deutlich erkennbare Fäden mit dem Durchmesser der Nadel ersichtlich waren, die sich nicht mit dem Wasser mischten. Unter Rühren konnten die Fäden zwar geteilt, aber nicht gelöst werden; wobei Bruchstücke der Fäden mit dem bloßen Auge erkennbar blieben. Bei Injektion eines derartigen Geles in Blutgefäße käme es zu Verstopfungen in den Gefäßen.

### Beispiele 4-7

Matrixtabletten mit folgender Zusammensetzung pro Tablette

| **Beispiel** | **4** | **5** | **6** | **7** |
|---|---|---|---|---|
| (-)-(1R,2R)-3-(3-Dimethylamino-1-ethyl-2-methylpropyl)phenol-Hydrochlorid | 100 mg | 100 mg | 100 mg | 100 mg |
| Hydroxypropylmethylcellulose (Metolose 90 SH 100.000 von Fa. Shinetsu), 100.000 mPa·s | 80 mg | 80 mg | 80 mg | 80 mg |
| Carboxymethylcellulose (Tylose C300) | 10 mg | | | |
| Carboxymethylcellulose (Tylose C600) | | 10 mg | | |
| Hydroxyethylcellulose (Tylose H300) | | | 10 mg | |
| Hydroxyethylcellulose (Tylose H4000) | | | | 10 mg |
| Mikrokristalline Cellulose (Avicel PH 102 von Fa. FMC) | 123 mg | 123 mg | 123 mg | 123 mg |
| Hochdisperses Siliciumdioxid | 4 mg | 4 mg | 4 mg | 4 mg |
| Magnesiumstearat | 3 mg | 3 mg | 3 mg | 3 mg |
| Gesamtmenge | 320 mg | 320 mg | 320 mg | 320 mg |

wurden wie in Beipiel 1 angegeben hergestellt.

Jeweils eine dieser Tabletten wurde gemörsert und mit 10ml Wasser geschüttelt. Es bildete sich eine viskose, trübe Suspension mit eingeschlossenen Luftblasen. Nach Absetzen der groben, festen Bestandteile der Suspension wurde das Gel in eine Spritze mit einer Nadel mit 0,9 mm Durchmesser aufgezogen. Das aufgezogene Gel wurde in 37°C warmes Wasser gespritzt, wobei deutlich sichtbare Fäden mit dem Durchmesser der Nadel erkennbar blieben, die sich nicht mit dem Wasser mischten. Unter Rühren konnten die Fäden zwar geteilt, aber nicht gelöst werden, wobei die Bruchstücke der Fäden mit dem bloßen Auge erkennbar blieben. Bei Injektion eines derartigen Gels in Blutgefäße käme es zu Verstopfungen in den Gefäßen.

### Beispiele 8-13 (Referenz)

Matrixtabletten mit folgender Zusammensetzung pro Tablette

| **Beispiel** | **8** | **9** | **10** | **11** | **12** | **13** |
|---|---|---|---|---|---|---|
| Morphinsulfat Pentahydrat | 60 mg | 60 mg | 60 mg | 60 mg | 60 mg | 60 mg |
| Hydroxypropylmethylcellu lose (Metolose 90 SH 15.000 von Fa. Shinetsu), 15.000 mPa·s | 60 mg | 60 mg | 60 mg | 60 mg | 60 mg | 60 mg |
| Xanthan, NF | 10 mg | 30 mg | | | | |
| Carboxymethylcellulose (Tylose C300) | | | 10 mg | | | |
| Carboxymethylcellulose (Tylose C600) | | | | 10 mg | | |
| Hydroxyethylcellulose (Tylose H300) | | | | | 10 mg | |
| Hydroxyethylcellulose (Tylose H4000) | | | | | | 10 mg |
| Mikrokristalline Cellulose (Avicel PH 102 von Fa. FMC) | 123 mg | 123 mg | 123 mg | 123 mg | 123 mg | 123 mg |
| Hochdisperses Siliciumdioxid | 4mg | 4 mg | 4 mg | 4 mg | 4 mg | 4 mg |
| Magnesiumstearat | 3 mg | 3 mg | 3 mg | 3 mg | 3 mg | 3 mg |

Jeweils eine dieser Tabletten wurde gemörsert und mit 10ml Wasser geschüttelt. Es bildete sich eine viskose, trübe Suspension mit eingeschlossenen Luftblasen. Nach Absetzen der groben, festen Bestandteile der Suspension wurde das Gel in eine Spritze mit einer Nadel mit 0,9 mm Durchmesser aufgezogen. Das aufgezogene Gel wurde in 37°C warmes Wasser gespritzt, wobei deutlich sichtbare Fäden mit dem Durchmesser der Nadel erkennbar bleiben, die sich nicht mit dem Wasser mischten. Unter Rühren konnten die Fäden zwar geteilt, aber nicht gelöst werden, wobei die Bruchstücke der Fäden mit dem bloßen Auge erkennbar blieben. Bei Injektion eines derartigen Gels in Blutgefäße käme es zu Verstopfungen in den Gefäßen.

### Beispiele 14-18 (Referenz)

Kapseln mit folgender Zusammensetzung der einfachen Pulvermischung pro Kapsel (Kapselgröße 4)

| **Beispiel** | **14** | **15** | **16** | **17** | **18** |
|---|---|---|---|---|---|
| Morphinsulfat Pentahydrat | 20 mg | 20 mg | 20 mg | 20 mg | 20 mg |
| Xanthan, NF | 10 mg | | | | |
| Carboxymethylcellulose (Tylose C300) | | 10 mg | | | |
| Carboxymethylcellulose (Tylose C600) | | | 10 mg | | |
| Hydroxyethylcellulose (Tylose H300) | | | | 10 mg | |
| Hydroxyethylcellulose (Tylose H4000) | | | | | 10 mg |
| Mikrokristalline Cellulose (Avicel PH 102 von Fa. FMC) | 68 mg | 68 mg | 68 mg | 68 mg | 68 mg |
| Hochdisperses Siliciumdioxid | 1 mg | 1 mg | 1 mg | 1 mg | 1 mg |
| Magnesiumstearat | 1 mg | 1 mg | 1 mg | 1 mg | 1 mg |

Jeweils eine dieser Tabletten wurde gemörsert und mit 10ml Wasser geschüttelt. Es bildete sich eine viskose, trübe Suspension mit eingeschlossenen Luftblasen. Nach Absetzen der groben, festen Bestandteile der Suspension wurde das Gel in eine Spritze mit einer Nadel mit 0,9 mm Durchmesser aufgezogen. Das aufgezogene Gel wurde in 37°C warmes Wasser gespritzt, wobei deutlich sichtbare Fäden mit dem Durchmesser der Nadel erkennbar blieben, die sich nicht mit dem Wasser mischten. Unter Rühren konnten die Fäden zwar geteilt, aber nicht gelöst werden, wobei die Bruchstücke der Fäden mit dem bloßen Auge erkennbar blieben. Bei Injektion eines derartigen Gels in Blutgefäße käme es zu Verstopfungen in den Gefäßen.

## Patentansprüche

1. Gegen parenteralen Mißbrauch gesicherte, feste Darreichungsform, **dadurch gekennzeichnet, dass** sie neben (1R*,2R*)-3-(3-Dimethylamino-1-ethyl-2-methyl-propyl)-phenol oder einem physiologisch verträglichen Salz davon wenigstens ein viskositätserhöhendes Mittel in solchen Mengen aufweist, dass das in einem mit Hilfe von 10 ml Wasser bei 25°C aus der Darreichungsform gewonnene Extrakt ein wirkstoffhaltiges, noch nadelgängiges Gel bildet, welches beim Einbringen mit einer Injektionsnadel mit einem Durchmesser von 0,9 mm in eine weitere Menge von 37°C warmem Wasser mindestens 1 Minute visuell unterscheidbar bleibt.

2. Darreichungsform gemäß Anspruch 1, **dadurch gekennzeichnet, dass** sie ein oder mehrere viskositätserhöhende Mittel ausgewählt aus der Gruppe bestehend aus mikrokristalliner Cellulose mit 11 Gew.-% Carboxymethylcellulose-Natrium, Carboxymethylcellulose-Natrium, Polyacrylsäure, Johannisbrotkernmehl, Citrus-Pectin, Wachsmaisstärke, Natriumalginat, Guarkernmehl, lota-Carrageen, Karaya Gummi, Gellangummi, Galaktomannan, Tarakernmehl, Propylenglykoalginat, Apfelpektin, Pektin aus Zitronenschale, Natrium-Hyaluronat, Tragant, Taragummi, fermentiertes Polysaccharid- Welan Gum und Xanthan-Gummi aufweist.

3. Darreichungsform gemäß Anspruch 1 oder 2, **dadurch gekennzeichnet, dass** sie die viskositätserhöhenden Mittel in einer Menge von ≥ 5 mg pro Darreichungsform, d.h. pro Dosiereinheit aufweist.

4. Darreichungsform gemäß einem der Ansprüche 1 bis 3 zur oralen Applikation.

5. Darreichungsform gemäß Anspruch 4, **dadurch gekennzeichnet, dass** sie in Form einer Tablette, einer Kapsel oder in Form eines oralen osmotischen therapeutischen Systems (OROS) vorliegt.

6. Darreichungsform gemäß Anspruch 4, **dadurch gekennzeichnet, dass** sie in multipartikulärer Form, vorzugsweise in Form von Mikrotabletten, Mikrokapseln, Mikropellets, Granulaten, Sphäroiden, Perlen oder Pellets, vorzugsweise in Kapseln abgefüllt oder zu Tabletten verpreßt, vorliegt.

7. Darreichungsform gemäß einem der Ansprüche 1 bis 6, **dadurch gekennzeichnet, dass** sie wenigstens (1R*,2R*)-3-(3-Dimethylamino-1-ethyl-2-methyl-propyl)-phenol oder ein physiologisch verträgliches Salz davon zumindest teilweise in retardierter Form aufweist.

8. Darreichungsform gemäß einem der Ansprüche 5 bis 7, **dadurch gekennzeichnet, dass** sie einen magensaftresistenten Überzug aufweist.

## Claims

1. A parenteral abuse-proofed solid administration form, **characterized in that**, in addition to (1R*,2R*)-3-(3-dimethylamino-1-ethyl-2-methylpropyl)phenol or a physiologically acceptable salt thereof, it comprises at least one viscosity-increasing agent in a quantity such that the extract obtained from the administration form with the assistance of 10 ml of water at 25 °C forms an active ingredient-containing gel which can still pass through a needle and which, when introduced with a hypodermic needle with a diameter of 0.9 mm into a further quantity of water at 37 °C, remains visually distinguishable for at least 1 minute.

2. The administration form according to claim 1, **characterized in that** it has one or more viscosity-increasing agents selected from the group consisting of microcrystalline cellulose with 11 wt.% carboxymethylcellulose sodium, carboxymethylcellulose sodium, polyacrylic acid, locust bean gum, citrus pectin, waxy maize starch, sodium alginate, guar gum, iota-carrageenan, karaya gum, gellan gum, galactomannan, tara gum, propylene glycol alginate, apple pectin, lemon peel pectin, sodium hyaluronate, tragacanth, tara gum, fermented polysaccharide welan gum and xanthan gum.

3. The administration form according to claim 1 or 2, **characterized in that** it comprises the viscosity-increasing agents in a quantity of ≥ 5 mg per administration form, i.e., per administration unit.

4. The administration form according to any one of claims 1 to 3 for oral application.

5. The administration form according to claim 4, **characterized in that** it assumes the form of a tablet, a capsule or of an oral osmotic therapeutic system (OROS).

6. The administration form according to claim 4, **characterized in that** it assumes a multiparticulate form, preferably the form of microtablets, microcapsules, micropellets, granules, spheroids, beads or pellets, preferably filled in capsules or compressed to form tablets.

7. The administration form according to any one of claims 1 to 6, **characterized in that** it has at least (1R*,2R*)-3-(3-dimethylamino-1-ethyl-2-methylpropyl)phenol or a physiologically acceptable salt thereof at least partially in controlled release form.

8. The administration form according to any one of claims 5 to 7, **characterized in that** it has an enteric coating.

## Revendications

1. Forme d'administration solide, sécurisée contre l'usage parentéral abusif, **caractérisée en ce qu'**elle présente en plus de (1R*,2R*)-3-(3-diméthylamino-1-éthyl-2-méthyl-propyl)-phénol ou d'un sel physiologiquement compatible de celui-ci, au moins un agent augmentant la viscosité en quantités telles que l'extrait obtenu à l'aide de 10 ml d'eau à 25°C à partir de la forme d'administration forme un gel encore injectable, contenant une substance active, lequel reste discernable visuellement pendant au moins 1 minute lors de l'introduction avec une aiguille d'injection d'un diamètre de 0,9 mm dans une autre quantité d'eau à 37°C.

2. Forme d'administration selon la revendication 1, **caractérisée en ce qu'**elle présente un ou plusieurs agents augmentant la viscosité choisis dans le groupe constitué de cellulose microcristalline avec 11 % en poids de carboxyméthylcellulose sodique, de carboxyméthylcellulose sodique, d'acide polyacrylique, de farine de caroube, de pectine de citrus, d'amidon de maïs cireux, d'alginate de sodium, de farine de guar, de carraghénane iota, de gomme karaya, de gomme gellane, de galactomannane, de farine de tara, d'alginate de propylène glycol, de pectine de pomme, de pectine d'écorce de citron, d'hyaluronate de sodium, d'astragale, de gomme tara, de gomme welan polysaccharide fermenté et de gomme xanthane.

3. Forme d'administration selon la revendication 1 ou 2, **caractérisée en ce qu'**elle présente l'agent augmentant la viscosité dans une quantité de ≥ 5 mg par forme d'administration, autrement dit par unité de dosage.

4. Forme d'administration selon l'une quelconque des revendications 1 à 3 pour l'application orale.

5. Forme d'administration selon la revendication 4, **caractérisée en ce qu'**elle se présente sous la forme d'un comprimé, d'une gélule ou sous la forme d'un système thérapeutique osmotique oral (OROS).

6. Forme d'administration selon la revendication 4, **caractérisée en ce qu'**elle se présente sous forme multiparticulaire, de préférence sous la forme de microcomprimés, de microgélules, de micropastilles, de granulés, de sphéroïdes, de perles ou de pastilles, de préférence remplie dans des gélules ou pressée en comprimés.

7. Forme d'administration selon l'une quelconque des revendications 1 à 6, **caractérisée en ce qu'**elle présente au moins du (1R*,2R*)-3-(3-diméthylamino-1-éthyl-2-méthyl-propyl)-phénol ou un sel physiologiquement compatible de celui-ci au moins en partie sous forme retardée.

8. Forme d'administration selon l'une quelconque des revendications 5 à 7, **caractérisée en ce qu'**elle présente un enrobage résistant au suc gastrique.
